# EUROPEAN PATENT APPLICATION

(11) **EP 4 545 032 A1**
(43) Date of publication of application: **30.04.2025**
(21) Application number: 23206191.1
(22) Date of filing: 26.10.2023
(51) Int. Cl.: A61B 18/18

(54) **A DEVICE FOR HAIR REMOVAL OR TREATMENT OF SKIN**

(71) Applicant: Koninklijke Philips N.V., 5656 AG Eindhoven (NL)
(72) Inventor: VAN ABEELEN, Frank Anton, 5656 AG Eindhoven (NL); BOAMFA, Marius Iosif, 5656 AG Eindhoven (NL); PALERO, Jonathan Alambra, 5656 AG Eindhoven (NL); BOURQUIN, Yannyk Parulian Julian, 5656 AG Eindhoven (NL)
(74) Representative: Philips Intellectual Property & Standards

(57) **Abstract**

A device for hair removal or treatment of skin generates both treatment light and skin pigmentation analysis light, which are delivered to the skin via a reflective housing. The skin pigmentation analysis light is detected after reflection by the skin and the detector is located outside the reflective housing. The reflective housing comprises a light escape opening or window, wherein no direct analysis light path through the light escape opening or window aligns with the detector. This reduces a signal offset caused by non-reflected light from the light source which generates the analysis light.

## Description

### FIELD OF THE INVENTION

This invention relates to a device for hair removal or treatment of skin, in particular by applying treatment light to the skin.

### BACKGROUND OF THE INVENTION

For hair removal, photo-epilation devices are well-known. People use hair epilators or depilators to remove unwanted hair. Typical target areas for women are the face, armpit, arm, leg, bikini line, and body. Men use light-based epilators also on the chest and back.

One common type of photo-epilation devices is based on the filtered output of a flashlamp, known as Intense Pulsed Light, IPL. It is known to provide home use photo-epilation devices with a safety feature of a skin pigmentation sensor that measures the skin tone of a specific user, or more specifically the melanin index. Melanin is the substance responsible for skin pigmentation. For example, IPL-based photo-epilation devices of Philips feature different treatment head attachments for treating different areas of the body, and each attachment features a skin pigmentation sensor.

Such a device can then provide advice that the IPL device is safe to use by the specific user (and block flashing if it is unsafe). A recommendation on safe device settings can then be provided or the device may be adapted automatically for safety.

A typical pigmentation sensor is based on measurement of the ratio of light reflected (or back-scattered) from the skin at two different wavelengths, e.g., 660 nm and 880 nm. This method makes use of the fact that the absorption coefficient of melanin decreases as a function of wavelength.

From data from several IPL studies, it has been found that the melanin index (MI) correlates well with lightness (L*) from the CIELAB color space as defined by the International Commission on Illumination (CIE). Thus, an alternative method for measuring the melanin index is based on measurement of the lightness of the reflected light.

The current solution for embedding a skin pigmentation sensor in the treatment head attachment is costly and results in a rather large area treatment head, which can limit the view of the user to the area being treated.

It would be desirable if a photo-epilation device could provide skin pigmentation sensing in the main body of the device and not in the treatment head attachment. However, a challenge in implementing a skin pigmentation sensor in the photo-epilation device is positioning of the detector and light source for the pigmentation sensor.

There are two contributions to the light signal received at the detector. Light reflected or backscattered from the skin forms the useful signal. In addition, there is light travelling directly from the source to the detector (either along a direct line of sight or via reflective surfaces, e.g., the glass envelope of the IPL flash lamp or the light guide around the lamp). This signal is also detected in the absence of skin, and it may be considered to form offset light. Unless the optics of the pigmentation sensor are completely separated from the path of the treatment light, which is unfavorable in cost and volume, the offset light tends to be of similar magnitude as the useful signal. The noise typically scales with the combined signal including both contributions. Therefore, the variance in the measured MI, which relates to the useful signal to noise ratio, exceeds an acceptable limit.

It would thus also be of interest to provide a photo-epilation device that provides skin pigmentation sensing with efficient and robust reduction of the offset contribution to the detected signal, thereby to provide accurate MI measurement with minimal noise.

### SUMMARY OF THE INVENTION

The invention is defined by the claims.

According to examples in accordance with an aspect of the invention, there is provided a device for hair removal or treatment of skin, comprising:
a light source system comprising a treatment part for generating treatment light and an analysis part for generating skin pigmentation analysis light;
a reflective housing within which the treatment light and skin pigmentation analysis light are delivered to the skin;
a detector for detecting the skin pigmentation analysis light after reflection by the skin, wherein the detector is located outside the reflective housing; and
a controller for controlling the light source system, wherein the controller is configured to determine a skin pigmentation from the detected skin pigmentation analysis light,
wherein the reflective housing comprises a light escape opening or window, wherein no direct paths from the analysis part of the light source system through the light escape opening or window align with the detector.

This device provides treatment light and analysis light (for determining skin pigmentation). A detector is used for the analysis of skin pigmentation. To reduce a signal offset, which deteriorates the signal to noise ratio, the arrangement avoids pigmentation analysis light reaching the detector before it has been reflected by the skin. This is achieved by routing the analysis light through an escape opening or window. The escape opening or window creates a direct light path between the skin and the detector, but there is no such direct path between the light source system, in particular the part that generates the skin pigmentation analysis light, and the detector. Note that the direct path may be a straight path through an opening, but an escape window may deflect the light (received directly from the analysis part of the light source system) by refraction or reflection.

A direct path is blocked from the light source system to the detector (i.e., the light source system does not overlap the path between the skin and the detector). This is achieved by selecting the light escape opening position and size and the position and size of the detector such that the angle between (i) the line of sight of the analysis part of the light source to the escape opening or window and (ii) the line of sight of the detector to the escape opening or window is more than the half of the analysis light source acceptance angle as defined by the escape opening.

It is noted that the reflection by the skin may include specular and diffuse reflection from the air-skin interface and also backscatter from deeper layers of the skin.

The reflective housing for example comprises a curved back reflector around the treatment part of the light source system at a side opposite to a skin contact region of the device and a reflective side wall between the curved back reflector and the skin contact region.

This is a known reflector system for an IPL device. The curved back reflector is for example a parabolic or partially parabolic reflector and the side wall is a tubular reflector. The tubular reflector may have a circular or rectangular or other cross-sectional shape.

The light escape opening or window is for example positioned:
at the reflective side wall; or
at an edge of the curved back reflector adjacent to the reflective side wall.

The treatment part and the analysis part of the light source system for example share the same light source. In this arrangement, there is only one light source, and it is controlled in an analysis mode and in a treatment mode.

In this case, the controller may be configured to control the (shared) light source to provide a pre-flash, before providing treatment light, for use as the pigmentation analysis light.

The shared light source of the light source system for example comprises a flash lamp and the device comprises a main capacitor arrangement and a booster capacitor arrangement for storing charge for use in delivering energy to the flash lamp, wherein the controller is configured to discharge the booster capacitor arrangement to provide the pre-flash. The capacitor charge provides the source for current through the lamp, wherein the electric energy is converted to light energy. This provides a simple way to implement the pre-flash function.

The detector may comprise first and second detector areas each with an associated narrowband filter. This enables a single wavelength, or broadband, light source to be used to provide measurements at two wavelengths.

The treatment part of the light source system for example comprises a first light source and the analysis part of the light source system comprises a second light source, and wherein the second light source is positioned outside the reflective housing, and the reflective housing comprises a light entry opening for receiving the pigmentation analysis light from the second light source.

In this set of examples, separate light sources are used so that they may be optimized for their respective functions. The second light source can then be positioned outside the reflective housing so that the reflective housing does not need to be increased in size.

The second light source may comprise two light source elements of different wavelengths, again to enable measurements at different wavelengths.

The light escape opening or window for example has a size and position such that light reflected from substantially the full skin area to be treated by the device is directly projected through the light escape opening or window onto the detector. For example at least 80% or at least 90% or all of the skin area may have a direct light path to the detector through the light escape opening.

In this way, the light escape opening or window includes the shape of the envelope of the set of light paths between the skin area to be treated and the detector. In one design, the light escape opening or window has a size and shape that corresponds to the shape of the envelope of the set of light paths. This means that only light from the skin has a direct path to the detector which gives optimal photon collection efficiency.

The device may further comprise a lens at the light escape opening or window. This enables a smaller opening or window to be used while maintaining the photon collection efficiency.

The the light escape opening or window may instead comprise an opening including a structured reflector for reflecting light received directly from the analysis part of the light source. In this way, a straight line path between the analysis part of the light source system and the detector may be allowed, but a structured opening or window deflects the light to prevent a direct path to the detector.

In all designs, a positioning angle between (i) a normal to the skin surface and (ii) a path from the center of the the skin treatment area to the detector is more than half the opening angle, in the same plane as the positioning angle, of the treatment-light beam incident on the skin. This gives a minimal specular reflection from the skin incident on the detector. Specular reflections do not contain information about the pigmentation level.

The controller may be configured to determine a skin pigmentation from:
an analysis of two wavelengths of skin pigmentation analysis light; or
an analysis of the lightness of the skin pigmentation analysis light.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an IPL device;
Fig. 2 shows a first example of a photo-epilation device 50 with an arrangement of the light source system and detector;
Fig. 3 shows a partial cross-section of the reflective housing around the flash lamp;
Fig. 4 shows a first alternative arrangement to that of Fig. 2;
Fig. 5 shows a second alternative arrangement to that of Fig. 2; and
Fig. 6 shows a second alternative arrangement to that of Fig. 2; and
Fig. 7 shows the angular distribution of the treatment light as it exits the device and enters the skin.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for hair removal or treatment of skin that generates both treatment light and skin pigmentation analysis light. The light is delivered to the skin via a reflective housing. The skin pigmentation analysis light is detected after reflection by the skin and the detector is located outside the reflective housing. The reflective housing comprises a light escape opening or window, wherein no direct analysis light path through the light escape opening or window aligns with the detector. This reduces a signal offset caused by non-reflected light from the light source which generates the analysis light.

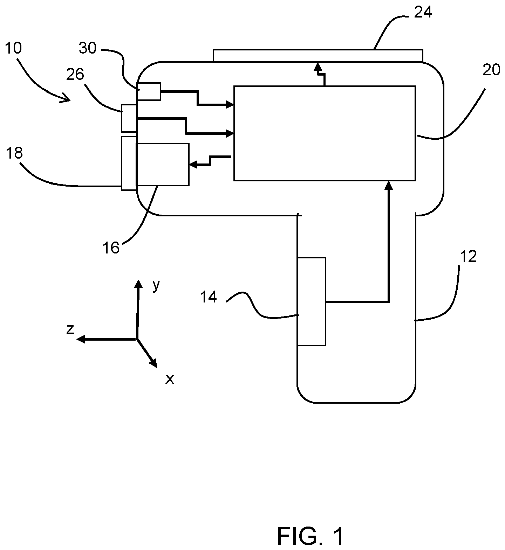

Fig. 1 shows an IPL device 10, in the form of a hand-held device having a handle 12 with a trigger 14, an IPL light source 16 providing light through a light delivery window 18. A controller 20 controls the light source 16 to deliver light flashes to the light delivery window 18.

The device of Fig. 1 also has an output interface 24, such as a screen and/or indicator lights and/or loudspeaker. The output interface to the user may instead or additionally be provided by a remote device with which the hand-held device communicates. A skin detection sensor 26 is used to detect skin contact. It may comprise a single sensor, or two or more sensors to detect that the light output window is flat against the skin.

In use of the device, a user applies the light output window to the skin and presses the trigger 14 to deliver a pulse of light. The user then slides the device over the skin to the next treatment location (or lifts the device and relocates it), and applies the trigger to deliver the next flash of light. It is also possible in some devices to keep the trigger depressed during the line treatment. It will then automatically flash every time a capacitor in the device is charged enough, which is typically 1-2 seconds depending on the setting. The user may then just move the device to the next spot, wait for the next flash, and move on again.

It is known to provide a skin pigmentation sensor 30 as part of the treatment device, in order to provide personalized recommendations and safety functions for the individual user.

The invention relates to an improved incorporation of skin pigmentation analysis into this type of device.

The device of the invention has a photo-epilation treatment unit, such as an intense pulsed light (IPL) light source, and a reflector with a reflecting inner surface for providing shielding of the intense pulsed light from leaking during use to avoid eye damage and to provide efficient guidance of the intense pulsed light to be incident on the target skin region.

The skin pigmentation sensing makes use of a light source (which may be the IPL light source or a separate light source used exclusively for analysis) and a detector. An escape window or opening is arranged in the wall of the reflector for providing an escape path for the backscattered light towards the detector, such that the detector is exposed to the backscattered light while minimizing the offset.

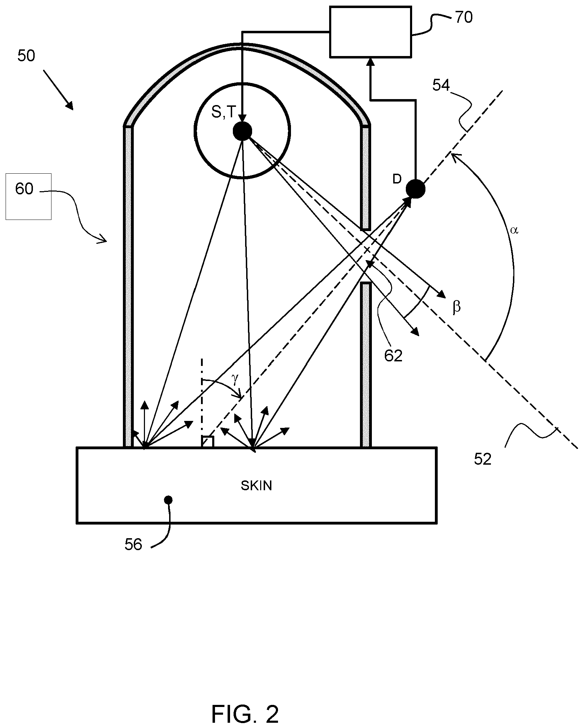

Fig. 2 shows a first example of a photo-epilation device 50 with a first example of an arrangement of the light source system and detector.

The light source system S,T in this example comprises a single light source, which combines a treatment part T for generating treatment light and an analysis part S for generating skin pigmentation analysis light. Even though they may be a single shared light source, they will be referred to separately below as the treatment light source and the analysis light source.

The light guiding system comprises a reflective housing 60 within which the treatment light and skin pigmentation analysis light are delivered to the skin. A detector D is for detecting the skin pigmentation analysis light after reflection and/or backscattering by the skin. The detector D is located outside the reflective housing.

A controller 70 is provided for controlling the light source system S,T and for determining a skin pigmentation from the detected skin pigmentation analysis light, in known manner.

The reflective housing 60 comprises a light escape opening or window 62 (which will simply be termed a "window" below). No direct paths from the light source system (and more specifically from the analysis light source S) align through the window with the detector D.

What is meant by "direct path" in this context is a light path from the analysis light source to the window 62, which then passes through the window. Such direct light paths have thus not interacted with the skin. Thus, there could be a straight line from the analysis light source S to the detector D through the window, but if the window provides a deflection (refraction or reflection) of the incident light such that it does not reach the detector, there is again no such direct path.

This device provides treatment light and analysis light (for determining skin pigmentation). By avoiding the direct paths as explained above, the problem of a signal offset is reduced. Pigmentation analysis light is prevented from reaching the detector before it has been reflected or backscattered by the skin.

Fig. 2 shows light path angles. The light path from the analysis light source S to the window 62 is along a general direction 52 with a (total) angular spread β to reach the boundary of the window 62. The light paths from the skin to the detector D through the window 62 are spread around a central path 54 at an angle γ to a normal to the skin surface. The angle between the two central paths 52, 54 is α.

A direct path is blocked from the analysis light source S to the detector.

In the example of Fig. 2, the window is an opening, so does not deflect or reflect light paths. In this case, the blocking is achieved by selecting the window position and size and the position and size of the detector D such that the angle α between (i) the line of sight 52 of the analysis light source to the window and (ii) the line of sight 54 of the detector to the window is more than the half of the analysis light source acceptance angle β/2.

In other examples, the window has a light processing function, so that the direct paths may not be straight lines and hence the relationships explained above do not apply.

More generally therefore, the position, size and structure of the window and the position of the detector D are configured such that light emitted from the analysis light source S onto the window is substantially blocked from reaching the detector D.

As mentioned above, the IPL flash lamp may be used as the analysis light source for pigmentation analysis. The pigmentation sensing should be performed in advance of the treatment flash, with the aid of a pre-flash.

For the pre-flash, the lamp may be operated at a low flash energy (e.g., < 5% of the energy of a treatment flash). The pre-pulse must be such that pigmentation can be sensed without the pulse being intense enough for photo-epilation and potentially inflicting skin damage if the skin is (too) dark.

Some known photo-epilation devices (the Philips Lumea device for example) have so-called booster capacitors in addition to the main capacitor. The booster capacitors support the ignition of the plasma in the lamp at the start of a flash. A pre-flash can be generated by discharging only the booster capacitors and not using the main capacitor. Alternatively, the main capacitor is used, but the discharge is cut-off after a fraction of the usual pulse length.

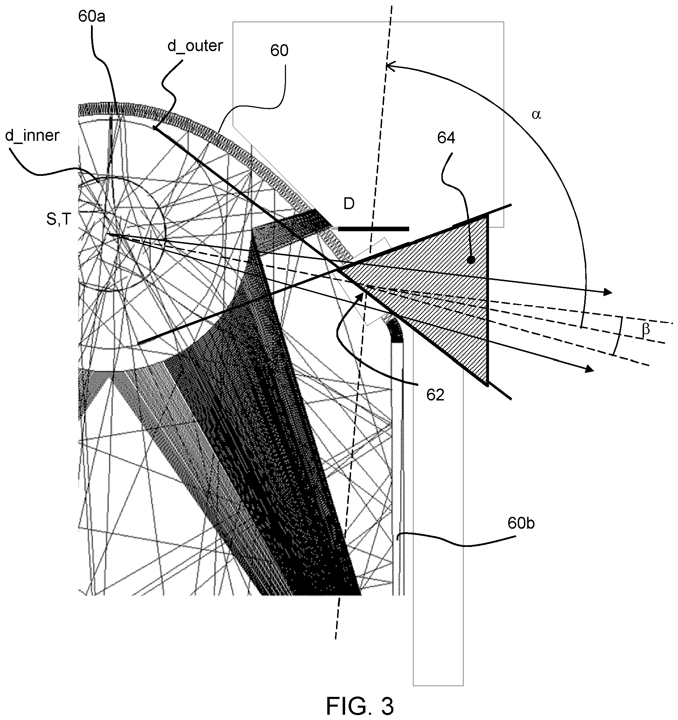

Fig. 3 shows a partial cross-section of the reflective housing 60 around the IPL flash lamp, which again functions as both the treatment light source T and analysis light source S. Behind the lamp, the light guide is closed by a curved (e.g., parabolic or partially parabolic) back reflector 60a. The back reflector reflects light emitted in the backward direction and creates a certain degree of collimation in the forward light beam. The back reflector is spaced from the skin by a tubular reflector 60b, for example with a rectangular or circular cross-sectional shape.

The flash lamp S,T for example has an inner diameter of 2 mm (d inner) and an outer diameter of 4 mm (d_outer). It is arranged in close proximity to the back reflector 60a.

The window 62 in this example is positioned in the back reflector, 60a just before the transition to the straight tubular part 60b of the reflector 60. Light originating from the flash lamp S,T and passing through the window will remain within the conic section 64 (i.e. a two dimensional shape in a single plane). The detector D is arranged outside this section.

The conic section 64 is shown for the light paths from the full outer diameter D_outer of the flash lamp S,T. This reduces the offset even further than the minimum requirement explained above.

In order to enable measurement of MI based on two wavelengths, the detector D may be provided with two sensitive areas, each equipped with a different narrow-band filter. Suitable values for the central wavelengths of these filters are 660 nm and 880 nm. For a lightness-based detection, no filters are needed.

The standard deviation in melanin index o_MI has been simulated with a detector placed directly behind an opening in the main reflector (facing inward) as representing the standard solution, and also with a detector positioned according to the invention. The source of noise in the case of a pre-flash from the IPL flash lamp is the spectral variability of the lamp which has been assumed to be 5% for this modeling.

For the standard solution, o_MI is found to be 156 whereas the solution of the invention enables o_MI to be reduced to 56, showing improved accuracy in the measurement of MI.

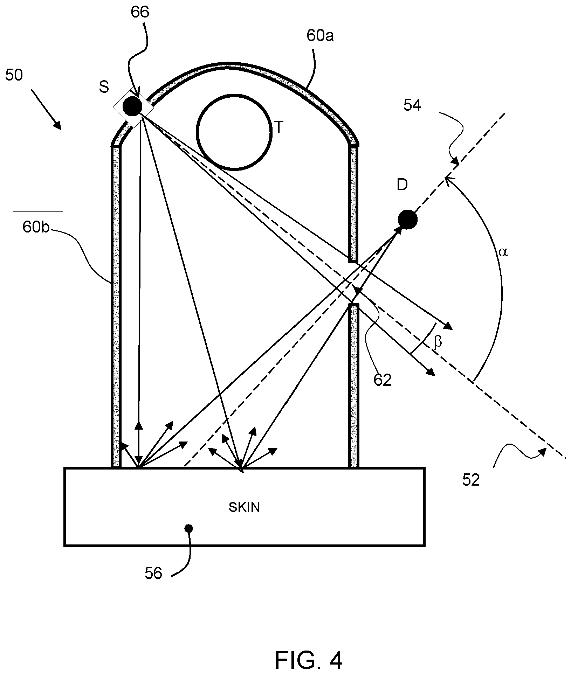

Fig. 4 shows an alternative solution in which the analysis light source is not the IPL flash lamp, so that there is a separate analysis light source S and treatment light source T.

In the example of Fig. 4, the window 62 is at the reflective side wall 60b. Furthermore, the analysis light source S is positioned outside the light guiding chamber defined by the reflector. Light enters the reflector from the analysis light source S through another window 66. However, the same angular conditions as explained above apply.

For the two-wavelength method of pigmentation measurement, the analysis light source S may comprise two LEDs with different peak wavelengths, e.g., 660 nm and 880 nm. The detector D only needs to have a single sensitive area. Alternatively, a broad-spectrum (white, blue + phosphor) LED may be used in combination with a detector with two sensitive areas equipped with different narrow-band filters.

A (solid) light guide such as fiber may be used between the analysis light source Sand the opening 66 in the light guiding system. This enables arrangement of the analysis light source S at a distance with minimal light loss. A sufficiently large air gap between the analysis light source S and the back reflector 60a can isolate the analysis light source from a high voltage that is applied to the reflector for ignition of the flash lamp.

Another means for electrical isolation is an isolating window (plus conductive layer against capacitive coupling) between the analysis light source S and the opening 66 in the back reflector.

The analysis light source S may instead be at the same side of the light guiding system as the window 62. In another variation, the analysis light source S and the detector D emit and receive light respectively from the same window 62. The analysis light source may be placed to direct light directly to the skin or via reflection from the reflector 60.

As mentioned above, a high ignition voltage is typically applied to the back reflector, such as an ignition voltage of about 15 kV. This may also damage the detector D which itself will be connected to PCB components near ground potential, due to electrical breakdown and/or capacitive current induction. Moreover, the temperatures of the back reflector and layer of air around the lamp envelope exceed 200 °C.

In combination with the main positioning requirement for the detector as explained above, the distance of the detector D to the back reflector 60a can be increased to reduce risks.

Optionally, the position and size of the window 62 and the position of the detector D are configured such that the detection collection angle ϕ results in optimal photon collection efficiency.

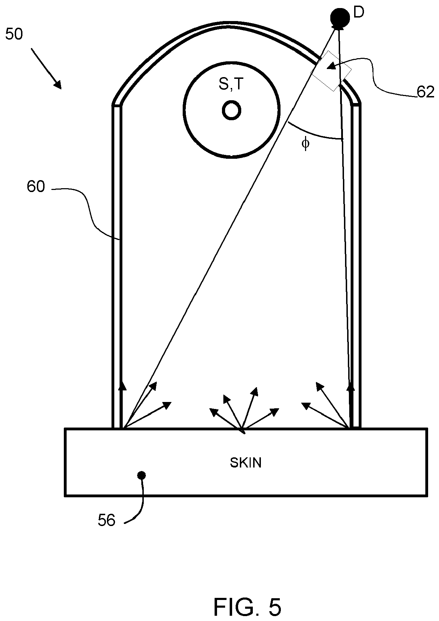

In Fig. 5, the angle of acceptance ϕ of the detector D is illustrated and shown to cover the entire treatment area. An additional advantage of this full acceptance angle is that the pigmentation is measured as an average over the entire treatment window, rather than as a value representative of a small spot only.

In order for the full skin area to be detected by the detector, the window 62 has a size and position such that light reflected from substantially the full skin area to be treated by the device is directly projected through the window onto the detector. The window may be larger than the envelope formed by those direct light paths to the detector.

However, in order to provide optimal photon efficiency while minimizing the contribution of stray light to the signal offset, the window may have a size and shape which matches the envelope formed by the direct light paths to the detector from the overall skin area. The smaller the size of the window, the smaller the chance that some light (e.g. reflected by a filter without antireflection coating) reaches the detector without being reflected by the skin. The envelope described above is the minimum size that still provides optimal photon efficiency. It means that only light from the skin has a direct path to the detector.

In the examples above, the window simply comprises an opening. However, a lens may be used at the window 62 in order to reduce the size of the window while maintaining the surface coverage.

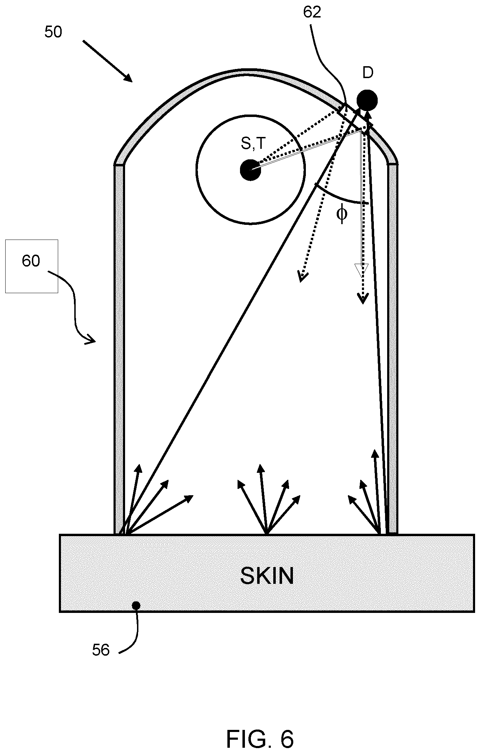

In another example shown in Fig. 6, the window 62 is a transparent window positioned in the back reflector but structured such that incident angles corresponding to direct light from the analysis light source S are not transmitted. The light scattered back from the skin is incident from different angles and is transmitted to the detector D.

Such an angular dependence of the transmission through the window can be created by (triangular) grooves, or a diffractive structure, on one of the surfaces of the window. Thus, a direct path to the detector is avoided by reflecting the light from the analysis light source, by total internal reflection. Alternatively, a grating may be used to prevent direct light from the analysis light source to escape from the window 62. The grating may for example be based on triangular grooves.

A photo-epilation device typically comprises a treatment spectral filter between the flash lamp and the skin, for optimal and safe photo-epilation treatment of skin. The surfaces of this filter back-reflect a fraction of incident light due to Fresnel reflection. These reflections form a virtual light source that contributes to the offset recorded by the detector.

Preferably, both sides of the spectral filter are coated with an appropriate anti-reflection coating to minimize this offset contribution. It is explained above that the detector configuration enables the standard deviation in melanin index, σ_MI, to be reduced from 156 to 56. Further modeling shows that the use of an antireflection coating on both sides of the filter enables a further reduction to 41.

The position and size of the window 62 and the position of the detector D may be configured such that the angle between the line of sight 54 of the detector D to the center of the window 62 and a normal to the skin surface, γ (see Fig. 2) results in minimal specular reflectance incident on the detector D.

In particular, the position and size of the window 62 and the position of the detector D is configured such that the angle between the line of sight 54 of the detector D to the center of the window 62 and a normal to the skin surface, y, is more than half the opening angle τ of the central collimated part of the beam incident on the skin.

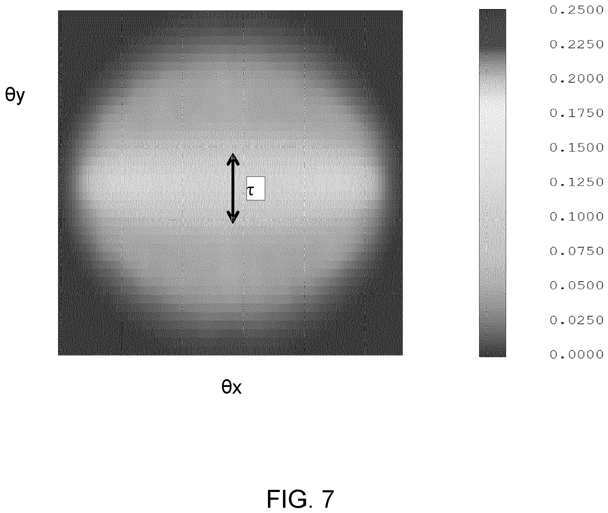

Fig. 7 shows the angular distribution of the treatment light as it exits the device and enters the skin (θₓ and 0_{y} are angles in the zx and zy planes where the z-axis is aligned with the optical axis of the device as represented in Fig. 1).

In this example, the axis of the back reflector extends in a direction perpendicular to the zy plane. As a result, the light beam is collimated in that plane to an opening angle τ (e.g. defined as full width half maximum).

A part of the light incident on the skin experiences specular reflection, wherein the exitant angle equals the incident angle. Specular reflections do not contain information about the pigmentation level. In particular, specular reflections that occur at the air-skin interface will be the same for light and dark skin. The difference between skin pigmentation levels only becomes apparent in light that has penetrated into the skin, to the location where the melanin resides, and has then been backscattered.

A detector positioned at an angle γ > τ (angles taken in the zy plane) will not see the specular reflections. In this way, specular reflections from the skin into the detector D are avoided.

Note that the acceptance angle of the detector may view the skin via a reflection in the side wall of the reflector rather than directly. Only light scattered by the skin, and thus containing relevant skin information, can reach the detector D. Especially for dark skin, this enhances the precision of the pigmentation measurement.

Alternatively, for the configuration of Fig. 4, the light may be cross-polarized to avoid specular reflection. This is achieved by using a polarized light source and detector or by placing a polarizer in front of the light source and detector.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A device for hair removal or treatment of skin, comprising:
a light source system (S,T) comprising a treatment part (T) for generating treatment light and an analysis part (S) for generating skin pigmentation analysis light;
a reflective housing (60) within which the treatment light and skin pigmentation analysis light are delivered to the skin;
a detector (D) for detecting the skin pigmentation analysis light after reflection by the skin (56), wherein the detector (D) is located outside the reflective housing (60); and
a controller (70) for controlling the light source system, wherein the controller is configured to determine a skin pigmentation from the detected skin pigmentation analysis light,
wherein the reflective housing (60) comprises a light escape opening or window (62), wherein no direct paths from the analysis part (S) of the light source system (S,T) through the light escape opening or window (62) align with the detector (D).

2. The device of claim 1, wherein the reflective housing comprises a curved back reflector (60a) around the treatment part of the light source system at a side opposite to a skin contact region of the device and a reflective side wall (60b) between the curved back reflector and the skin contact region.

3. The device of claim 2, wherein the curved back reflector (60a) is a parabolic or partially parabolic reflector and the side wall is a tubular reflector.

4. The device of any one of claims 2 to 3, wherein the light escape opening or window (62) is positioned:
at the reflective side wall; or
at an edge of the curved back reflector adjacent to the reflective side wall.

5. The device of any one of claims 1 to 4, wherein the treatment part (T) and the analysis part (S) of the light source system share a common light source.

6. The device of claim 5, wherein the controller (70) is configured to control the common light source to provide a pre-flash, before providing treatment light, for use as the pigmentation analysis light.

7. The device of claim 6, wherein the light source system comprises a flash lamp and the device comprises a main capacitor arrangement and a booster capacitor arrangement for storing charge for use in delivering energy to the flash lamp, wherein the controller is configured to discharge the booster capacitor arrangement to provide the pre-flash.

8. The device of any one of claims 5 to 7, wherein the detector (D) comprises first and second detector areas each with an associated narrowband filter.

9. The device of any one of claims 1 to 4, wherein the treatment part (T) of the light source system comprises a first light source and the analysis part of the light source system comprises a second light source (S), and wherein the second light source (S) is positioned outside the reflective housing, and the reflective housing comprises a light entry opening (66) for receiving the pigmentation analysis light from the second light source (S).

10. The device of claim 9, wherein the second light source (S) comprises two light source elements of different wavelengths.

11. The device of any one of claims 1 to 10, wherein the light escape opening or window (62) has a size and position such that light reflected from substantially the full skin area to be treated by the device is directly projected through the light escape opening or window (62) onto the detector (D).

12. The device of any one of claims 1 to 11, further comprising a lens at the light escape opening or window.

13. The device of any one of claims 1 to 12, wherein the light escape opening or window (62) comprises an opening including a structured reflector for reflecting light received directly from the analysis part of the light source system.

14. The device of any one of claims 1 to 13, wherein a positioning angle (γ) between (i) a normal to the skin surface and (ii) a path from the center of the the skin treatment area to the detector is more than half the opening angle (τ), in the same plane as the positioning angle (γ), of the treatment-light beam incident on the skin.

15. The device of any one of claims 1 to 14, wherein the controller is configured to determine a skin pigmentation from:
an analysis of two wavelengths of skin pigmentation analysis light; or
an analysis of the lightness of the skin pigmentation analysis light.
